# EUROPÄISCHE PATENTANMELDUNG

(11) **EP 4 595 999 A1**
(43) Veröffentlichungstag der Anmeldung: **06.08.2025**
(21) Anmeldenummer: 24181418.5
(22) Anmeldetag: 11.06.2024
(51) Int. Cl.: A61M 5/142, A61M 39/28

(54) **MEDIZINISCHES INFUSIONSSYSTEM UND ZUGEHÖRIGES BETRIEBSVERFAHREN**

(30) Priorität: 01.02.2024 EP 24155333
(71) Anmelder: B. Braun Melsungen AG, 34212 Melsungen (DE)
(72) Erfinder: Schwarz, Jan, 34212 Melsungen (DE)
(74) Vertreter: Tergau & Walkenhorst Intellectual Property GmbH

(57) **Zusammenfassung**

Medizinisches Infusionssystem mit
• mindestens einem zum Einlegen in oder Anschließen an einem Infusionsgerät (16), insbesondere einer Peristaltikpumpe oder einer Spritzenpumpe, vorgesehenen Einmalartikel (4), insbesondere einem Infusionsschlauch bzw. einer Spritze, der entweder selbst oder durch ein mit ihm verbundenes Zubehörteil mit einem maschinenlesbaren Code versehen ist, welcher eine Ablaufdauer, insbesondere eine Verweildauer oder eine Haltbarkeitsdauer oder ein Verfallsdatum, des Einmalartikels (4) beinhaltet oder repräsentiert,
• mindestens einem Infusionsgerät (16) mit einer Aufnahme (30) oder einem Anschluss für den Einmalartikel (4), ferner mit einem Sensor zur Erfassung des Codes, und ferner mit einer Pumpensteuerung (24), die derart ausgelegt oder programmiert ist, dass sie anhand des erfassten Codes die Ablaufdauer, insbesondere die Verweildauer, die Haltbarkeitsdauer oder das Verfallsdatum, des Einmalartikels (4) ermittelt.

## Beschreibung

Die Erfindung betrifft ein medizinisches Infusionssystem mit mindestens einem zum Einlegen in oder Anschließen an ein Infusionsgerät, insbesondere eine Infusionspumpe oder eine Spritzenpumpe, vorgesehenen Einmalartikel, insbesondere einem Infusionsschlauch oder einer Spritze, sowie mit einem zugehörigen Infusionsgerät. Die Erfindung betrifft weiterhin ein Betriebsverfahren für ein Infusionsgerät.

Die Verwendung von Einmalartikeln wie Infusionsschläuchen im medizinischen Umfeld ist gängige Praxis. Jedoch ist die Kontrolle und Gewährleistung der Nichtüberschreitung der Haltbarkeit oder des Verfallsdatums solcher Artikel von entscheidender Bedeutung, um mögliche Risiken für den Patienten zu minimieren.

Beispielsweise ist ein Infusionsschlauch für ein medizinisches Infusionssystem nach seiner Sterilisation abgepackt für eine bestimmte Zeit, für gewöhnlich 5 Jahre, haltbar. Gleichzeitig ist auch die Einsatzdauer, und damit eine Verweildauer in einem Infusionsgerät üblicherweise begrenzt. So kann bspw. der Einsatz eines Infusionsschlauches in einer Infusionspumpe, insbesondere in einem peristaltischen Kaubereich, über die Zeit zu einer Ermüdung des Schlauchmaterials und ggf. zu einem Risiko der Sterilität oder zu einer abweichenden Fördergenauigkeit führen. Auch kann es, abhängig von dem in den Einmalartikel eingebrachten Lösungen, nach Inbetriebnahme des Infusionsgeräts und Einsetzen des Einmalartikels, sinnvoll sein, eine maximale Verendungsdauer oder Verweildauer zu bestimmen, um sicherzustellen, dass ein Einmalartikel nur für eine solch vorbestimmte Zeit in dem Infusionsgerät verwendet werden kann. Nach Ablauf der Ablaufdauer, insbesondere der Verweildauer, der Haltbarkeitsdauer oder einem Verfallsdatum, darf der Infusionsschlauch nicht mehr verwendet werden. Bisweilen wird diese Vorgabe aber - absichtlich oder unabsichtlich - missachtet.

Die vorliegende Erfindung zielt darauf ab, dieses Problem zu lösen, indem sie ein Infusionssystem und ein zugehöriges Verfahren bereitstellt, das die Überwachung der Ablaufdauer, insbesondere der Verweildauer, der Haltbarkeit oder des Verfallsdatums von Einmalartikeln während des Anlagenbetriebs ermöglicht. Die Erfindung strebt einerseits danach sicherzustellen, dass nur Einmalartikel mit einer gültigen, noch nicht überschrittenen Haltbarkeit oder einem Verfallsdatum verwendet werden, wodurch die Patientensicherheit und die Wirksamkeit der medizinischen Behandlung verbessert werden. Andererseits ist es Ziel der Erfindung sicherzustellen, dass Einmalartikel nur bis zum Erreichen der maximalen Verweildauer verwendet werden, wodurch ebenfalls die Patientensicherheit und die Wirksamkeit der medizinischen Behandlung verbessert werden. Es soll dabei verstanden werden, dass im Rahmen der Erfindung die Haltbarkeit oder ein Verfallsdatum zum einen und/oder eine maximale Verweildauer zum anderen jeweils einzeln, wahlweise unabhängig voneinander oder gleichzeitig erfasst werden kann.

Die Aufgabe wird erfindungsgemäß gelöst durch ein medizinisches Infusionssystem mit den Merkmalen des Anspruchs 1. Das zugehörige Verfahren ist in Anspruch 11 definiert.

Demnach ist ein medizinisches Infusionssystem vorgesehen mit
- mindestens einem zum Einlegen in oder Anschließen an einem Infusionsgerät, insbesondere einer Peristaltikpumpe oder einer Spritzenpumpe, vorgesehenen Einmalartikel, insbesondere einem Infusionsschlauch bzw. einer Spritze, der entweder selbst oder durch ein mit ihm verbundenes Zubehörteil mit einem maschinenlesbaren Code versehen ist, welcher eine Ablaufdauer, insbesondere eine Verweildauer oder eine Haltbarkeitsdauer oder ein Verfallsdatum, des Einmalartikels beinhaltet oder repräsentiert,
- mindestens einem Infusionsgerät mit einer Aufnahme oder einem Anschluss für den Einmalartikel, ferner mit einem Sensor zur Erfassung des Codes, und ferner mit einer Pumpensteuerung, die derart ausgelegt oder programmiert ist, dass sie anhand des erfassten Codes die Ablaufdauer, insbesondere die Verweildauer, die Haltbarkeitsdauer oder das Verfallsdatum, des Einmalartikels ermittelt.

Der Begriff "Ablaufdauer", wie hierin verwendet, bezeichnet bevorzugt eine Zeitspanne, insbesondere eine vorbestimmte Zeitspanne, mit deren Ablauf ein Einmalartikel nicht mehr verwendet werden soll.

Dabei ist insbesondere umfasst, dass der Begriff "Ablaufdauer" in einer ersten Alternative bevorzugt die Haltbarkeitsdauer oder ein Verfallsdatum meint. In diesem Zusammenhang bezieht sich der Begriff "Ablaufdauer" bevorzugt auf die gesamte Dauer ab Herstellung des Einmalartikels bis zu dem Zeitpunkt, an dem der Einmalartikel unabhängig von dessen Lagerung oder Verwendung nicht mehr verwendet werden sollte. Mit anderen Worten bezeichnet der Begriff "Ablaufdauer" in der ersten Alternative bevorzugt ein Mindest-Haltbarkeitsdatum des Infusionseinmalartikels. Dieses kann besonders bevorzugt mit farbigen Klemmen, ähnlich einer Art Güte-Siegel oder TÜV-Siegel angesehen werden.

Zusätzlich oder alternativ meint der Begriff "Ablaufdauer" wie hierin verwendet in einer zweiten Alternative bevorzugt eine Verweildauer, also eine maximale Zeitspanne, die der Einmalartikel ggf. mit Unterbrechungen innerhalb des Infusionsgeräts verwendet werden darf. Gemäß dieser zweiten Alternative ist eine Verweildauer bevorzugt eine maximale Gesamt-Nutzungsdauer bei laufender Förderung/Therapie. Dabei ist die Verweildauer gemäß der zweiten Alternative üblicherweise deutlich kürzer im Vergleich zur Haltbarkeitsdauer gemäß erster Alternative. Es soll dabei verstanden werden, dass im Rahmen der Erfindung die Haltbarkeit oder ein Verfallsdatum im Sinne einer maximalen Gesamt-Nutzungsdauer zum einen und/oder eine maximale Verweildauer zum anderen jeweils einzeln, wahlweise unabhängig voneinander oder gleichzeitig erfasst werden kann.

In beiden Fällen, der ersten und der zweiten Alternative, kann die Ablaufdauer bevorzugt abhängig von dem Material des Einmalartikels und/oder abhängig von dem im Einmalartikel enthaltenen Lösungen, sowie ggf. auch der bestimmungsgemäßen Verwendung vorbestimmt sein. Für gewöhnlich ist der Einmalartikel, also bspw. eine Spritze oder ein Schlauch, zunächst ungefüllt und wird erst kurz vor der Anwendung mit einer Flüssigkeit befüllt, insb. bspw. eine Spritze, oder an einen Infusionsbeutel, bspw. bei einem Schlauch, angeschlossen. Die Erfindung ist jedoch darauf nicht beschränkt und kann zusätzlich oder alternativ auch vorbefüllte Artikel umfassen, wobei sich dann eine Ablaufdauer auch auf die Verweildauer und/oder die Haltbarkeitsdauer der Befüllung beziehen kann.

In einer bevorzugten Ausführungsform des erfindungsgemäßen medizinischen Infusionssystems, ist die Pumpensteuerung derart ausgelegt oder programmiert, dass sie anhand des erfassten Codes die Ablaufdauer, insbesondere die Verweildauer, die Haltbarkeitsdauer oder das Verfallsdatum, des Einmalartikels ermittelt und mit einer Systemzeit vergleicht und je nach Ergebnis des Vergleichs ein Signal an den Benutzer erzeugt, das bevorzugt dazu geeignet ist einen Infusionsbetrieb mit dem Einmalartikel zu blockieren oder zuzulassen und, bevorzugt im Fall einer Blockade, weiterbevorzugt das Signal als eine Warnmeldung über eine Benutzerschnittstelle ausgibt.

Vorteilhafterweise ist die Pumpensteuerung derart ausgelegt oder programmiert, dass sie im Fall der Blockade eine Warnmeldung, etwa ein akustisches Signal und/oder eine Anzeige auf einem Display, über eine Benutzerschnittstelle ausgibt.

In einem bevorzugten Anwendungsfeld ist der Einmalartikel ein Infusionsschlauch, und das Zubehörteil ist eine den Fluss von Infusionslösung durch den Infusionsschlauch regulierende Schiebeklemme oder sonstige Schlauchklemme, insbesondere eine "Anti-Free-Flow-Klemme", die beim Einlegen in die Infusionspumpe durch eine die Aufnahme abdeckende Verschlussklappe oder einen ähnlichen Mechanismus betätigt wird. Insbesondere kann aus Gründen der Sicherheit daran gedacht sein, dass eine Klemme manuell eingelegt, beim Öffnen der Frontklappe mittels eines Mechanismus zu lösen, bspw. einem Haken, herausgezogen, ist, um freiem Fluss bei der Öffnung der Frontklappe zu vermeiden.

In bevorzugter Ausgestaltung ist der Code in Form einer Farbkodierung auf dem Einmalartikel oder dem Zubehörteil ausgebildet, und der zugehörige Sensor ist ein Farbsensor. Vorteilhafterweise ist zum Zweck der Auswertung in dem Infusionssystem, insbesondere in der Pumpensteuerung eine Zuordnungstabelle abgespeichert, die die verschiedenen Farben der Farbkodierung mit einer jeweiligen Ablaufdauer, insbesondere einer Verweildauer, einer Haltbarkeitsdauer oder einem Verfallsdatum, verknüpft. Eine solche Zuordnungstabelle kann in einer vorteilhaften Ausführungsform zusätzlich oder alternativ auch außerhalb der Pumpensteuerung im oder sogar außerhalb des Infusionsgeräts hinterlegt sein. Beispielsweise kann eine Zuordnungstabelle in einer Cloud und/oder auf externen Speichermedien oder Servern hinterlegt sein. Bevorzugt ist die Zuordnungstabelle digital signiert und/oder verschlüsselt abgespeichert.

Alternativ kann der Code ein graphischer Code, insbesondere ein 2D-Code oder ein 3D-Code sein oder durch ein RFID-Tag oder durch eine Ausformung (mechanische Codierung) verwirklicht sein. Der Sensor zur Erfassung bzw. Auslesung des Codes ist dann selbstredend an die Art des Codes angepasst.

Indem die Pumpensteuerung derart ausgelegt oder programmiert ist, dass sie die Systemzeit mit einer externen Zeit synchronisiert, ist eine korrekte und präzise Überwachung der Haltbarkeit oder des Verfallsdatums des Einmalartikels gewährleistet, selbst in Umgebungen, in denen die interne Systemzeit der Infusionspumpe potenziell fehleranfällig oder nicht präzise genug ist.

Die zur Vorrichtung genannten Merkmale, Varianten, Aufgaben und Vorteile übertragen sich analog auf das Verfahren und umgekehrt.

Die Erfindung betrifft zusammenfassend ein Konzept zur Kodierung der Ablaufdauer, insbesondere der Verweildauer, der Haltbarkeitsdauer bzw. des Verfallsdatums eines Einmalartikels, insbesondere in Gestalt eines Infusionsschlauches oder einer Spritze, in Verbindung mit einer Infusionspumpe bzw. Spritzenpumpe, wobei die Kodierung und das Infusionsgerät zusammen sicherstellen, dass der Einmalartikel bzw. der Infusionsschlauch oder die Spritze nach Ablauf der Ablaufdauer, insbesondere der Verweildauer, der Haltbarkeitsdauer oder des Verfallsdatums nicht mehr verwendbar ist.

Die Kodierung des Einmalartikels kann insbesondere durch eine farbige Schiebeklemme bzw. eine farbige Markierung an der Schiebeklemme, welche dem Einmalartikel zugeordnet und vorzugsweise mit ihm verbunden ist, erfolgen.

Alternativ kann bspw. die Kodierung des Einmalartikels durch ein farbiges Bauteil, bspw. einem Spritzenkolben, bzw. eine farbige Markierung an dem Spritzenkolben, welches dem Einmalartikel zugeordnet und vorzugsweise mit ihm verbunden ist, erfolgen.

Die Farbe wird von dem Infusionsgerät, bzw. der Infusionspumpe oder der Spritzenpumpe, durch einen integrierten Senor erkannt. Innerhalb des Infusionsgeräts ist eine bevorzugt signierte und damit vor Veränderung geschützte Farbtabelle hinterlegt, die in Abhängigkeit von der Farbe eine Ablaufdauer, insbesondere eine Verweildauer, eine Haltbarkeitsdauer oder ein Verfallsdatum, jeweils plus ggf. eine Karenzzeit (für toleriertes Überschreiten der Ablaufdauer) definiert, in der der Infusionsschlauch innerhalb der Infusionspumpe ordnungsgemäß eingesetzt und verwendet werden kann. Ist das Ablaufdatum bzw. die Ablaufdauer überschritten, stellt die Infusionspumpe, bevorzugt durch Anzeige an den Nutzer, sicher, dass der Infusionsschlauch aufgrund der Überschreitung der Ablaufdauer, also der Verweildauer, des Verfallsdatums oder Haltbarkeitsdatums, nicht mehr nutzbar ist.

Zusätzlich bietet dieses Konzept die Möglichkeit, den Infusionsschlauch vor Nachahmung zu schützen, weil seine Kompatibilität zur Infusionspumpe einerseits durch das Verfallsdatum und andererseits durch die farbige Schiebeklemme kodiert sind. Wenn die Farbkodierung durch den Hersteller des Infusionsschlauches für Außenstehende unbekannt ist, ergibt sich somit ein Kopierschutz.

Zur praktischen Umsetzung des Konzepts, ist eine Infusionspumpe mit einem Sensor und einem in geeigneter Software und/oder Hardware implementierten Algorithmus zur Erkennung einer farbigen Schiebeklemme ausgestattet. Ein farbiger Markierungsbereich an der Schiebeklemme reicht dazu aus; es muss nicht die gesamte Schiebeklemme eingefärbt sein. Die Infusionspumpe ist damit in der Lage, eine unterschiedliche Anzahl an farbigen Schiebeklemmen zu erkennen. Die farbigen Schiebeklemmen werden somit genutzt, um eine Ablaufdauer, insbesondere Verfallsdaten des Infusionsschlauches zu kodieren; ähnlich wie bei einer TÜV-Plakette zur Hauptuntersuchung für Kraftfahrzeuge. So kann beispielsweise eine grüne Klemme in den Jahren 2023 bis 2028 genutzt werden; eine rote in den Jahren 2024 bis 2029 etc.

Das Verfallsdatum lässt sich in Abhängigkeit der farbigen Schiebeklemme separat definieren, wobei diese Definition zweckmäßigerweise in Form einer Lookup- bzw. Mappingtabelle innerhalb der Infusionspumpe hinterlegt ist (d. h. das Verfallsdatum ist in Abhängigkeit der Farbe unterschiedlich definierbar, ggf. mit einer Karenzzeit). Wird der Infusionsschlauch in die Infusionspumpe eingelegt, so wird die Farbe der Schiebeklemme von der Infusionspumpe erkannt. Aus den Parametern "erkannte Farbe" (Markierung der Schiebeklemme), der Mappingtabelle und anhand des aktuellen Datums, das der Infusionspumpe bekannt ist, beurteilt die Infusionspumpe, ob der eingelegte Infusionsschlauch nutzbar oder sein Haltbarkeitsdatum überschritten bzw. das Verfallsdatum erreicht ist. Im ersten Fall (Verfallsdatum nicht erreicht, d. h. Schlauch nutzbar) kann der Infusionsschlauch wie gewohnt von der Infusionspumpe genutzt werden, im zweiten Fall (Verfallsdatum erreicht oder überschritten) lehnt die Infusionspumpe den eingelegten Infusionsschlauch ab und blockiert die Nutzung dieses Einmalartikels, wodurch die Sicherheit gegenüber einem Patienten erhöht wird.

Auf ähnliche Weise können farbige Schiebeklemmen genutzt werden, um eine Ablaufdauer in Form einer Verweildauer des Infusionsschlauches zu kodieren; ähnlich einer Parkscheibe für Kraftfahrzeuge. So kann beispielsweise eine gelbe Klemme eine Gesamtnutzungsdauer von 96 Stunden angeben, eine orange Klemme kann eine Gesamtnutzungsdauer 5 Stunden bezeichnen etc.

Die Verweildauer lässt sich in Abhängigkeit der farbigen Schiebeklemme separat definieren, wobei diese Definition zweckmäßigerweise in Form einer Lookup- bzw. Mappingtabelle innerhalb der Infusionspumpe hinterlegt ist (d. h. die Verweildauer kann in Abhängigkeit der Farbe unterschiedlich definierbar, ggf. mit einer Karenzzeit). Wird der Infusionsschlauch in die Infusionspumpe eingelegt, so wird die Farbe der Schiebeklemme von der Infusionspumpe erkannt. Aus den Parametern "erkannte Farbe" (Markierung der Schiebeklemme), der Mappingtabelle und anhand des aktuellen Datums, das der Infusionspumpe bekannt ist, beurteilt die Infusionspumpe, ob der eingelegte Infusionsschlauch nutzbar oder seine Gesamtverweildauer überschritten ist. Hierbei ist es bevorzugt, dass die Pumpe, bzw. ein geeignetes Rechenmedium bspw. die Steuereinheit, eine bereits abgelaufene Zeit speichert und von Gesamt-Verweildauer subtrahiert, so dass eine resultierende restliche Verweildauer bestimmt wird und die Nutzung des Systems entweder zugelassen wird, oder blockiert wird. Dabei kann bevorzugt ein Signal an den Benutzer, insbesondere die Anzeige der restlichen Verweildauer als Signal an den Benutzer, ausgegeben werden kann. Im ersten Fall (Gesamtverweildauer nicht erreicht, d. h. Schlauch nutzbar) kann der Infusionsschlauch wie gewohnt von der Infusionspumpe genutzt werden, im zweiten Fall (Gesamtverweildauer erreicht oder überschritten) lehnt die Infusionspumpe den eingelegten Infusionsschlauch ab und blockiert die Nutzung dieses Einmalartikels, wodurch die Sicherheit gegenüber einem Patienten erhöht wird.

Die Lookup- bzw. Mappingtabelle ist in der Infusionspumpe vorteilhafterweise in einer signierten, d. h. verschlüsselten Form hinterlegt. Dadurch wird sichergestellt, dass sie vor Veränderungen geschützt ist und eine Veränderung ausschließlich durch autorisiertes Personal des Herstellers möglich ist.

Des Weiteren lässt sich die farbige Schiebeklemme in Verbindung mit dem Infusionsschlauch und der Infusionspumpe als Kopierschutz verwenden. Dieses Konzept bietet die Möglichkeit, den Infusionsschlauch vor Nachbau bzw. Nachahmung zu schützen, weil seine Kompatibilität zur Infusionspumpe einerseits durch das Verfallsdatum und andererseits durch die farbige Schiebeklemme kodiert sind. Wenn die Spezifikation der Farbkodierung (sprich die Mappingtabelle) durch den Hersteller des Infusionsschlauches für Außenstehende unbekannt ist, ergibt sich somit ein Kopierschutz.

Verschiedene Ausführungsbeispiele der Erfindung werden nachfolgend anhand der beigefügten Zeichnungen näher erläutert. Es zeigt in jeweils schematischer Weise:
- FIG. 1: in rein schematischer Darstellung eine Infusionspumpe mit eingelegtem Infusionsschlauch,
- FIG. 2: eine Schiebeklemme für einen Infusionsschlauch in Draufsicht,
- FIG. 3: in konkreterer Darstellung ein Infusionssystem mit einer als peristaltischen Pumpe (Schlauchpumpe) ausgebildeten Infusionspumpe, in die ein Infusionsschlauch eingelegt ist, und
- FIG. 4: ein Infusionssystem mit einer Spritzenpumpe.

Gleiche oder gleichartige Elemente sind in allen Figuren mit denselben Bezugszeichen versehen.

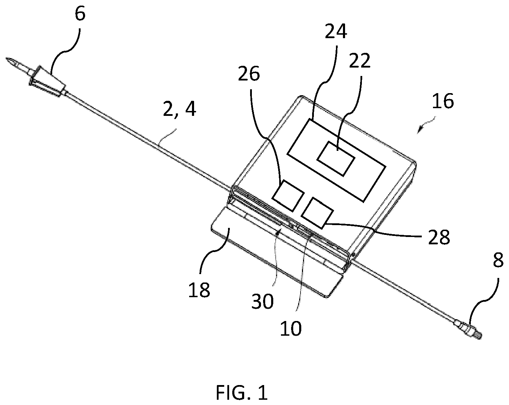

Ein Infusionsschlauch 2, wie er schematisch in FIG. 1 als Beispiel für einen Einmalartikel 4 innerhalb eines Infusionssystems dargestellt ist, ist ein medizinisches Gerät, das verwendet wird, um Flüssigkeiten wie Medikamente, Elektrolytlösungen oder Nährstoffe direkt in den Körper eines Patienten einzuleiten. Er spielt eine wichtige Rolle bei der intravenösen Therapie, indem er eine Verbindung zwischen der Infusionsquelle, insbesondere einem Infusionsbeutel mit Infusionslösung, und dem Patienten herstellt. Im Zusammenspiel mit einer Infusionspumpe 16 ermöglicht ein Infusionsschlauch 2 eine präzise Dosierung und Verabreichung von Substanzen, um den Gesundheitszustand des Patienten zu verbessern oder aufrechtzuerhalten.

Ein Infusionsschlauch 2 hat zwei Enden, die für unterschiedliche Zwecke ausgelegt sind:
Das Tropfkammer- oder Infusionsnadel-Ende 6: Hier ist üblicherweise eine Tropfkammer angeschlossen, in der die Infusionslösung abtropft und in den Körper des Patienten weiterfließt. Dieses Ende des Infusionsschlauchs 2 ist demnach in der Regel über eine Tropfkammer mit einer Infusionsnadel oder einem Infusionskatheter verbunden, die/der direkt in eine Vene eingeführt wird und somit in den Blutkreislauf des Patienten führt.

Das Infusionsbeutel- oder Infusionspumpen-Ende 8: Dieses Ende des Infusionsschlauchs 2 ist mit einem Infusionsbeutel oder einer Infusionspumpe 16 verbunden. Der Infusionsbeutel oder die Infusionspumpe 16 enthält oder liefert die Infusionslösung und reguliert den Fluss durch den Infusionsschlauch 2 in die Tropfkammer.

Zusammenfassend erfüllen die beiden Enden eines Infusionsschlauchs 2 unterschiedliche Funktionen: Das eine Ende ist mit der Infusionsquelle verbunden, während das andere Ende den direkten Zugang zum Patienten ermöglicht, um die Infusionsflüssigkeit in den Körper zu leiten.

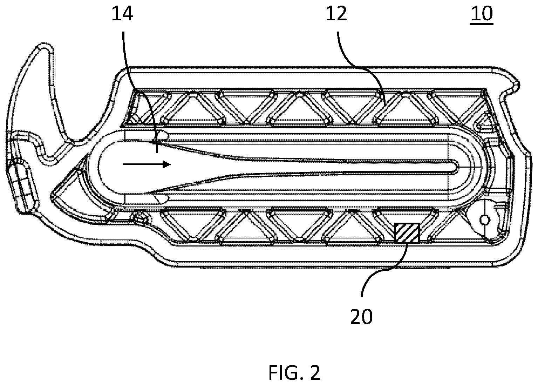

Eine in FIG. 2 dargestellte Schiebeklemme 10 ist ein wichtiges Zubehörteil im Zusammenhang mit einem Infusionsschlauch 2. Die Schiebeklemme 10 besteht normalerweise aus Kunststoff und wird auf den Infusionsschlauch 2 geschoben, um ihn bedarfsweise zu quetschen und dadurch den Fluss der Flüssigkeit zu unterbinden bzw. zu unterbrechen.

Eine Schiebeklemme 10 ist in FIG. 2 beispielhaft in detaillierter Draufsicht dargestellt. Sie dient nach dem oben Gesagten der Quetschung des Schlauchs und verhindert den freien Fluss (Free-Flow) der Infusionslösung. Die Schiebeklemme 10 weist einen flachen Grundkörper oder Rahmen 12, typischerweise aus Kunststoff, auf, welcher ein sich hier von rechts nach links verbreiterndes Langloch 14 umschließt. Die Schiebeklemme 10 ist im Betrieb auf den Infusionsschlauch 2 geschoben und umschließt ihn, so dass der Infusionsschlauch 2 das Langloch 14 durchquert. Wenn der Infusionsschlauch 2 im breiten Ende des Langlochs 14 angeordnet ist, wird er nicht gequetscht und hat seinen maximalen Querschnitt (Öffnungsstellung). Wird der Infusionsschlauch 2 hingegen relativ zur Schiebeklemme 10 in Pfeilrichtung zum schmalen Ende des Langlochs 14 hin verschoben, wird er zunehmend gequetscht und schließlich komplett verschlossen (Schließstellung).

Ein Infusionsschlauch 2 der genannten Art kann insbesondere zum Einlegen in eine als peristaltische Pumpe ausgebildete Infusionspumpe 16 ausgelegt sein. Bei einer peristaltischen Pumpe wird der Infusionsschlauch 2 periodisch durch rotierende Walzen oder Rollen oder auch durch Stößel, die an einer Welle angebracht sind, zusammengedrückt und entspannt, um die Flüssigkeit zu fördern. Der Vorteil einer derartigen Pumpe liegt darin, dass die Flüssigkeit im Schlauch bleibt und nicht mit der Pumpenmechanik in Berührung kommt, was die Kontamination der Flüssigkeit verhindert.

Eine auf den Infusionsschlauch 2 oder kurz Schlauch aufgeschobene Schiebeklemme 10 kann als sogenannte "Anti-Free-Flow-Klemme" oder "Free-Flow-Protection-Clamp" ausgebildet sein. Das ist eine Sicherheitsklemme oder kurz Klemme, die einen freien Fluss der zu fördernden Flüssigkeit bei der Entnahme aus dem Gerät (d. h. der Infusionspumpe 16) verhindern soll. Im Auslieferzustand des Schlauchsets ist die Klemme geöffnet. Diese muss nämlich während der Lagerung geöffnet sein, da sich durch längere Lagerung der Schlauch deformieren kann oder der Kunststoffeinmalartikel plastisch verformt. Beim Einlegen in das Gerät ist die Klemme daher zunächst geöffnet. Beim Entnehmen des Einmalartikels aus dem Gerät, zieht die Klappe per Öse, die in einen Haken der Klemme eingehakt wird, die Klemme wieder zu und schließt den Schlauch gegen Free-Flow. Deshalb wird die Klemme auch Free-Flow-Protection-Clamp genannt.

Zu diesem Zweck kann die Infusionspumpe 16 eine passend geformte, durch eine Verschlussklappe 18 verschließbare Aufnahme 30 (in FIG. 1 durch die Verschlussklappe 30 verdeckt) für den Infusionsschlauch 2 und die Schiebeklemme 10 aufweisen, wobei durch das Auf- und Zuklappen der Verschlussklappe 18 (alternativ durch das Einlegen oder Entnehmen des Infusionsschlauchs 2 mit der Schiebeklemme 10) die Schiebeklemme 10 angesteuert wird, wie oben beschrieben. Die Öse zum Herausziehen der Schiebeklemme 10 ist bevorzugt in der Frontklappe (Verschlussklappe) der Infusionspumpe 16 angebracht, was jedoch in der rein schematischen FIG. 1 nicht sichtbar ist.

Die Infusionspumpe 16 besitzt zudem üblicherweise eine pumpenseitige Klemme, die beim Einlegen des Schlauches den Schlauch quetschen und damit ebenfalls freien Fluss verhindern kann.

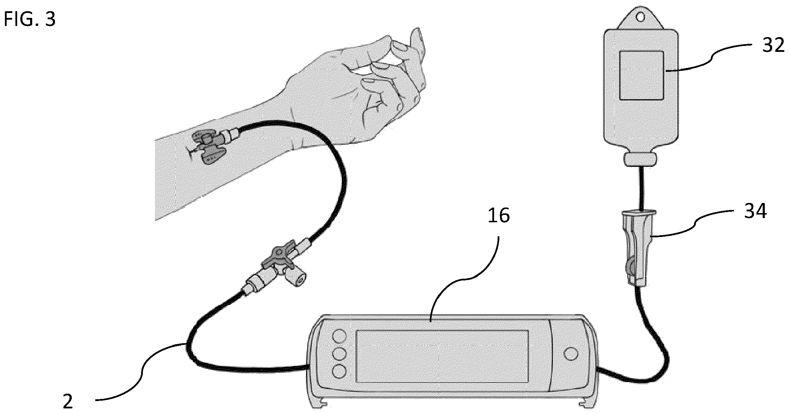

Mit einer Schiebeklemme 10 ist es möglich, den Infusionsschlauch 2 außerhalb der Infusionspumpe 6 mit einem Infusionsbeutel 32 zu verbinden, wobei durch die in der Schließstellung befindliche Schiebeklemme 10 ein Fluss von Infusionslösung verhindert wird. Das ist jedoch nur ein Nebeneffekt. Die eigentliche Schlie-βung erfolgt in der Regel durch eine Rollenklemme 34, wie in FIG. 3 dargestellt. Dort ist das Infusionssystem mit Infusionsbeutel 32, Infusionsschlauch 2, Rollenklemme 34 und Infusionspumpe 16 im Überblick dargestellt, wobei der Infusionsschlauch 2 mit der Schiebeklemme 10 in die Infusionspumpe 16 eingelegt (aufgrund der geschlossenen Verschlussklappe ist die Schiebklemme 10 hier nicht sichtbar).

Zur Inbetriebnahme wird der Infusionsschlauch 2 in einem dafür vorgesehenen Abschnitt zusammen mit der Schiebeklemme 10 bei geöffneter Verschlussklappe 18 in die Infusionspumpe 16 eingelegt und die Verschlussklappe 18 geschlossen. Dabei verschiebt sich die Schiebeklemme 10 in die Öffnungsstellung (wobei die Infusionspumpe 10 nun den Fluss von Flüssigkeit durch den Infusionsschlauch 2 steuert). Mit anderen Worten legt der Nutzer die Klemme in eine Aufnahme mit einem Klemmenmodul ein, wodurch sie geöffnet ist/wird. Teilweise gelingt dies auch durch das Schließen der Frontklappe, da der mechanische Druck die Klemme verschiebt.

Der Infusionsschlauch 2 mit bereits aufgesetzter Schiebeklemme 10 wird normalerweise als vormontierte Einheit im sterilisierten Zustand in einer sterilen bzw. hermetisch verschlossenen Verpackung aufbewahrt und zur Verwendung vorgehalten. Nach Ablauf einer vorgegebenen Ablaufdauer, insbesondere die Verweildauer, die Haltbarkeitsdauer oder das Verfallsdatum (gerechnet beispielsweise ab Produktions- oder Verpackungsdatum), ggf. unter Berücksichtigung einer tolerierten Überschreitung oder Karenz, oder nach Überschreiten eines vorgegebenen Verfallsdatums sollte der Infusionsschlauch 2 nicht mehr verwendet werden, weil dann beispielsweise die Sterilität des Verpackungsguts und/oder gewünschte Materialeigenschaften nicht mehr garantiert werden können. Gleichzeitig ist es auch nach der Inbetriebnahme und dem Einsetzen des Einmalartikels notwendig, die Ablaufdauer mit Hinblick auf die gesamte Dauer der Nutzung, also die Verweildauer zu begrenzen, die der Artikel ggf. mit Unterbrechungen in dem Infusionsgerät im Einsatz ist, da bedingt bspw. durch die im Einmalartikel enthaltenen Lösungen, Materialermüdungen, bspw. durch ein peristaltisches Pumpen, oder andere Gegebenheiten, die risikofreie Verwendung nicht mehr sichergestellt werden kann. Leider wird diese Vorgabe in der Praxis bisweilen ignoriert oder unabsichtlich missachtet.

Um solch eine unerwünschte Verwendung eines Infusionsschlauchs 2 nach Ablauf der Ablaufdauer, insbesondere der Verweildauer, der Haltbarkeitsdauer oder des Verfallsdatums zu verhindern, ist in einer bevorzugten Ausgestaltung die mit dem Infusionsschlauch 2 verbundene oder verbindbare und im oben beschriebenen Sinne als "Anti-Free-Flow-Klemme" zur Wechselwirkung mit einer zugehörigen Infusionspumpe 16 ausgebildete Schiebeklemme 10 an einer vorbestimmte Stelle mit einem maschinenlesbaren Code versehen / ausgestattet / beschriftet oder ausgebildet, der die Haltbarkeitsdauer oder das Verfallsdatum enthält oder repräsentiert.

Ein Code ist im Allgemeinen eine systematische Anordnung von Zeichen, Symbolen, Farben, Signalen oder anderen Informationsträgern, die dazu dienen, Informationen zu speichern, zu übermitteln, zu verschlüsseln, zu entschlüsseln oder zu verbergen. Ein Code ermöglicht die Speicherung und Übertragung von Informationen in einer strukturierten und/oder kodierten (insbesondere verschlüsselten) Form.

In bevorzugter Ausgestaltung handelt es sich bei dem Code um eine Farbkodierung 20, etwa in Gestalt einer (insbesondere einfarbigen) Farbmarkierung oder eines (insbesondere mehrfarbigen) Farbmusters an einer vorbestimmten Stelle am Rahmen 12 der Schiebeklemme 10. Die zur Kodierung und Kennzeichnung der Schiebeklemme 10 verwendete Farbe kann beispielsweise als Farbaufdruck, als Beschichtung, durch Anbringung eines Farbelements oder durch geeignete Wahl des Grundmaterials erzeugt sein.

Dabei ist in einer zugehörigen Zuordnungstabelle 22 (Lookup- bzw. Mappingtabelle) oder Farbtabelle eine idealerweise eineindeutige Zuordnung zwischen Farbe und Ablaufdauer, mit Hinblick auf die Haltbarkeitsdauer und das Verfallsdatums des Infusionsschlauchs 2 definiert, wobei zur Vereinfachung und zur Reduktion der notwendigen unterscheidbaren Farben bestimmte Gruppen oder Kategorien gebildet sein können; in einer sehr einfachen Form zum Beispiel: rot - haltbar bis Ende 2025, grün - haltbar bis Ende 2027, blau - haltbar bis Ende 2029.

Gleichzeitig oder alternativ kann in einer solchen zugehörigen Zuordnungstabelle 22 (Lookup- bzw. Mappingtabelle) oder Farbtabelle eine idealerweise eineindeutige Zuordnung zwischen Farbe und Ablaufdauer, mit Hinblick auf eine Verweildauer des Infusionsschlauchs 2 im Sinne einer maximale Verwendungsdauer als Ablaufdauer definiert werden. Auch hier können zur Vereinfachung und zur Reduktion der notwendigen unterscheidbaren Farben bestimmte Gruppen oder Kategorien gebildet sein; in einer sehr einfachen Form zum Beispiel: rosa - Gesamtnutzungsdauer 96 Stunden, grau - Gesamtnutzungsdauer 5 Stunden.

Es ist auch im Rahmen der vorliegenden Erfindung, dass mittels einer solchen Farbkodierung mehrere Parameterkombinationen gleichzeitig in einer zugehörigen Zuordnungstabelle 22 (Lookup- bzw. Mappingtabelle) oder Farbtabelle eindeutig zuordenbar hinterlegt sind, um Beispiel:
Gelb - Gesamtnutzungsdauer 96 Stunden, haltbar bis Ende 2025,
Orange - Gesamtnutzungsdauer 5 Stunden, haltbar bis Ende 2025,
hellblau -Gesamtnutzungsdauer 96 Stunden, haltbar bis Ende 2027,
Dunkelblau - Gesamtnutzungsdauer 96 Stunden bis, haltbar Ende 2029

In der Praxis können je nach verwendetem Sensortyp zur Farberkennung (siehe unten) verschiedene Farben nach ihren Eigenschaften Farbton, Sättigung und/oder Helligkeit (oder alternativen Charakterisierungen) unterschieden werden.

Die Zuordnungstabelle 22 ist vorteilhafterweise signiert und/oder verschlüsselt in der Pumpensteuerung 24 der Infusionspumpe 16 oder einer sonstigen, mit der Infusionspumpe 16 kommunizierenden Auswertungseinheit abgespeichert und damit einerseits vor einfacher Auslesbarkeit und anderseits vor einfacher Manipulierbarkeit durch unbefugte Personen geschützt.

Die Infusionspumpe 16, in die der Infusionsschlauch 2 mit der Schiebeklemme 10 zur Benutzung eingelegt wird, ist mit Mitteln für eine automatisierte, maschinelle Erfassung und Dekodierung / Entschlüsselung des auf der Schiebeklemme 10 angebrachten bzw. angeordneten Codes ausgestattet. Unter Verwendung der oben erwähnten Zuordnungstabelle 22, die in einer Speichereinheit der Pumpen-steuerung 24 (oder Auswertungseinheit) hinterlegt bzw. abgespeichert ist, ermittelt ein in der Pumpensteuerung 24 implementierter Algorithmus aus der im Code enthaltenen Information die Haltbarkeitsdauer oder das Verfallsdatum des Infusionsschlauchs 2 und vergleicht sie/es mit der aktuellen Systemzeit, die zuvor zumindest einmalig mit der externen Zeit abgeglichen wurde und vorteilhafterweise regelmäßig mit ihr synchronisiert wird. Zusätzlich oder alternativ kann eine Verweildauer des Einmalartikels erfasst und ggf. bereits abgelaufene Einsatzzeit, gespeichert werden. Ergibt dieser Vergleich, dass die Ablaufdauer, insbesondere die Verweildauer, die Haltbarkeitsdauer oder das Verfallsdatum, ggf. unter Einbeziehung einer vorgegebenen Karenz, überschritten wurde, so kann die Pumpensteuerung 24 bspw. über eine Benutzerschnittstelle ein Signal an den Benutzer ausgeben, das bevorzugt dazu geeignet ist einen Infusionsbetrieb mit dem Einmalartikel 4 zu blockieren oder zu zulassen. Wahlweise kann eine Ausgabe des Signals an die Benutzerschnittstelle bzw. den Nutzer unterbleiben. Mit anderen Worten kann zum Beispiel der weitere Betrieb erlaubt werden ohne, dass der Benutzer darüber besonders informiert wird. Zusätzlich oder alternativ kann das Signal weitere Informationen für den Benutzer umfassen. Beispielsweise kann das Signal eine Warnmeldung an den Benutzer sein. Auch kann das Signal eine verbleibende Ablaufdauer sein, insbesondere eine verbleibende Rest-Verweildauer oder eine Zeitspanne bis hin zur Haltbarkeitsdauer oder einem Verfallsdatum, Insbesondere kann das Signal eine Warnmeldung oder eine Aufforderung zum Schlauchwechsel sein und die Pumpensteuerung 24 verweigert oder blockiert oder stoppt den Betrieb mit dem alten Schlauch. Andernfalls kann der Betrieb normal weitergeführt werden.

Die beschriebene Überprüfung kann beispielsweise immer nach einem Wechsel des Infusionsschlauchs 2 bzw. nach einem solchen Wechsel anzeigenden Öffnen / Wiederverschließen der Verschlussklappe 18 durchgeführt werden. Im laufenden Betrieb hingegen wird sie zweckmäßigerweise nicht durchgeführt, um eine laufende Infusion nicht durch Abbruch oder Blockade zu gefährden. Gleichzeitig kann auch im laufenden Betrieb die Ablaufdauer in Form der maximalen Verweildauer regelmäßig oder mitlaufend überwacht werden.

Es kann auch in der Pumpensteuerung 24 vorgesehen sein, dass ein autorisierter Benutzer, etwa nach Eingabe eines Berechtigungscodes über ein Tastenfeld oder nach sonstiger Autorisierung, in der Lage ist, eine Blockade aufzuheben, etwa um in Notfallsituationen (z. B. Katastrophenfall oder Krieg mit entsprechenden Versorgungsengpässen) eine Infusion mit einem veraltetem Infusionsschlauch (über Ablaufdatum, insbesondere Verfallsdatum) durchzuführen. Ebenso kann ggf. vorgesehen sein, einen Artikel auch über die maximale Verweildauer hinaus nutzen zu können.

Zur Erkennung der Farbe der Farbkodierung 26 ist zweckmäßigerweise ein optischer Sensor, insbesondere ein Farbsensor 26 mit zugehöriger Beleuchtungseinheit 28 in die Infusionspumpe 16 integriert. Beispielsweise können die Komponenten derart angeordnet sein, dass von der Beleuchtungseinheit 28 emittiertes Licht auf die in der Aufnahme 30 sitzende Schiebeklemme 10 fällt und von dieser reflektiert wird, wobei das reflektierte Licht auf den Farbsensor 16 fällt. Der Farbsensor wertet dann beispielsweise ein Reflektionsspektrum aus. Alternativ kann bei entsprechender Anordnung der Komponenten ein Transmissionsspektrum ausgewertet werden, sofern das die Farbkodierung 26 tragende Element hinreichend lichtdurchlässig ist. In beiden Fällen (Reflektion oder Transmission) kann im Prinzip anstelle einer Beleuchtung durch eine Beleuchtungseinheit 28 eine Beleuchtung durch Umgebungslicht erfolgen, was jedoch wegen der schwankenden Umgebungslichtverhältnisse nicht immer praktikabel und zumindest fehleranfälliger ist. Anstelle von und/oder zusätzlich zu einer herkömmlichen Farbe im sichtbaren Bereich des optischen Spektrums kann auch eine spektrale Eigenschaft eines Emissionsspektrums und/oder Transmissionsspektrums im nicht-sichtbaren Spektralbereich zur Kennzeichnung der Schiebeklemme 10 und zur Kodierung der Haltbarkeitsdauer oder des Verfallsdatums verwendet werden. Dementsprechend kann die Beleuchtungseinheit 28 beispielsweise für die Emission von IR- oder UV-Licht ausgelegt sein. Entsprechendes gilt für die spektrale Sensitivität des Farbsensors 16, die an die interessierende Wellenlänge angepasst ist. Auch die Anregung und Auswertung von Fluoreszenzeffekten als Träger der Kodierung ist denkbar.

Der Farbsensor kann so beschaffen sein, dass er die Farbe der Farbkodierung ermittelt und dem Auswertungsalgorithmus oder der Auswertungseinheit der Pumpensteuerung mitteilt. Es kann aber auch so sein, dass nur gewisse physikalische Eigenschaften des reflektierten oder transmittierten Lichts durch den Farbsensor gemessen werden, wobei die eigentliche Auswertung und Bestimmung der Farbe dann in der Pumpensteuerung 28 oder Auswertungseinheit erfolgt. Anstelle einer in die Infusionspumpe 16 integrierten Auswertungseinheit kann auch eine Ausgestaltung vorgesehen sein, bei der die entsprechende Funktionalität zumindest teilweise in eine externe Komponente ausgelagert ist und beispielsweise durch einen Zentralrechner oder Cloudservice bereitgestellt wird.

In einer Abwandlung des beschriebenen Konzepts kann die zur Festlegung der Ablaufdauer, insbesondere der Verweildauer, der Haltbarkeitsdauer oder des Verfallsdatums verwendete Farbkodierung 26 an anderer Stelle als auf/an einer Schiebeklemme 10 angeordnet sein. Möglich ist beispielsweise eine mit dem Infusionsschlauch 2 verbundene oder verbindbare Markierungsfahne oder sonstige Kennzeichnung oder ein anderes entsprechendes Zubehörteil. Des Weiteren ist ein Farbaufdruck oder eine Farbschicht oder eine materialbedingte Farbkodierung direkt auf der Oberfläche des Infusionsschlauchs 2 eine mögliche Alternative.

Anstelle einer Farbkodierung 26 lassen sich auch auf dem Infusionsschlauch 2, der Schiebeklemme 10 oder einem anderen Zubehörteil aufgedruckte oder anderweitige angebrachte oder angeordnete Zeichencodes verwenden, die durch die Infusionspumpe 16 über einen optischen Sensor lesbar / auswertbar sind und damit die oben beschriebene Funktion abbilden. Insbesondere kann es zur Bestimmung einer Ablaufdauer im Sinne einer maximalen Verweildauer, zweckdienlich sein mittels einer entsprechenden Codierung den Einmalartikel eindeutig identifizierbar zu machen, um sicherzustellen, dass die Ablaufdauer des spezifischen Einmalartikels korrekt erfasst werden kann. Generell bezieht sich der Begriff "Zeichencode" auf jede Art von kodierter Darstellung von Informationen, die auf visuellen Elementen basiert, sei es durch spezielle Symbole, grafische Muster oder andere visuelle Markierungen. Insbesondere kommen Barcodes und QR-Codes als Informationsträger in Betracht: Dies sind Arten von Zeichencodes, die visuelle Muster verwenden, um Informationen darzustellen. Barcodes bestehen aus einer Reihe von parallelen Linien unterschiedlicher Breite, während QR-Codes typischerweise aus einem Muster von quadratischen Blöcken bestehen. Beide werden durch sensorbasierte Lesegeräte erfasst und in digitale Daten umgewandelt.

Zum Erschweren der Lesbarkeit und Manipulierbarkeit durch Unbefugte kann eine nicht-normierte oder nicht-standardkonforme (hausinterne) Kodierung verwendet werden.

Als weitere Alternative lassen sich RFID-Tags, welche die Ablaufdauer, insbesondere die Verweildauer, die Haltbarkeitsdauer oder das Verfallsdatum des Infusionsschlauchs 2 in kodierter Form enthalten, in den Infusionsschlauch 2, die Schiebeklemme 10 oder das sonstige Zubehörteil integrieren. Die auf dem RFID-Tag hinterlegten Informationen lassen sich durch einen geeigneten Sensor der Infusionspumpe 16 auslesen und, wie oben beschrieben, auswerten und nutzen. Der RFID-Tag kann auch eine dedizierte Kopierschutzsequenz enthalten. Der Infusionsschlauch 2 wird jedoch durch den RFID-Tag in seiner Herstellung teurer, da er in den Infusionsschlauch 2 integriert werden muss.

Anstelle einer Schiebeklemme 10, die durch Verschiebung relativ zum Infusionsschlauch 2 den Fluss von Flüssigkeit durch den Infusionsschlauch 2 reguliert, kann jede andere Schlauchklemme mit äquivalenter Funktion zum Einsatz kommen, etwa eine Dreh- oder Klappklemme. Auch in diesem Fall sind zweckmäßigerweise die Aufnahme 30 und/oder Verschlussklappe 18 der Infusionspumpe 16 derart beschaffen, dass sie die Schlauchklemme beim Einlegen in die und/oder Entnehmen aus der Infusionspumpe 16 in die Öffnungs- oder Schließstellung bringen.

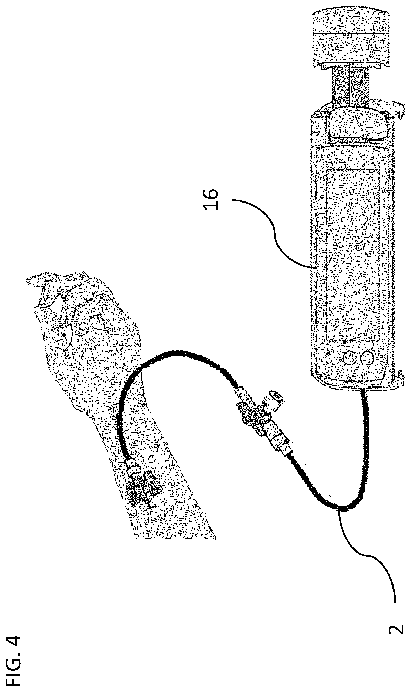

Anstelle eines Infusionsschlauchs 2 kann als Einmalartikel 4 auch ein Infusionsbeutel oder eine Spritze zum Anschließen oder Einlegen in/an die Infusionspumpe 16 vorgesehen und entsprechend mit einer Farbkodierung 20 oder sonstigen Codierung ausgestattet sein. Das heißt, alle weiter oben am Beispiel einer peristaltischen Pumpe beschriebenen Konzepte zur automatischen Erfassung und Berücksichtigung eine Ablaufdauer, insbesondere einer Verweildauer, einer Haltbarkeitsdauer oder eines Verfalldatums mittels codierter Schlauch- bzw. Schiebeklemme lassen sich sinngemäß auf eine Spritzenpumpe übertragen, wobei in diesem Fall vorzugsweise eine entsprechend codierte medizinische Spritze, die in die Pumpe eingelegt wird, die Ansteuerung bewirkt. Diese Variante ist in FIG. 4 veranschaulicht. Dabei ist es bevorzugt, dass der Spritzenkolben und/oder der Spritzenhohlkörper den Code trägt und insbesondere farbig ausgestaltet oder farbig markiert ist.

Eine andere Bezeichnung für eine Spritzenpumpe ist eine Kolbenpumpe. Im Gegensatz zur peristaltischen Pumpe, die durch Quetschen und Freigeben eines flexiblen Schlauches arbeitet, verwendet die Spritzenpumpe einen Kolbenmechanismus (innerhalb der Spritze), um Flüssigkeit zu fördern. Dabei bewegt sich ein Kolben in einem Zylinder vor und zurück, um Flüssigkeit anzusaugen und zu fördern.

### Bezugszeichenliste

- 2: Infusionsschlauch
- 4: Einmalartikel
- 6: Tropfkammer- oder Infusionsnadel-Ende
- 8: Infusionsbeutel- oder Infusionspumpen-Ende
- 10: Schiebeklemme
- 12: Rahmen
- 14: Langloch
- 16: Infusionspumpe
- 18: Verschlussklappe
- 20: Farbkodierung
- 22: Zuordnungstabelle
- 24: Pumpensteuerung
- 26: Farbsensor
- 28: Beleuchtungseinheit
- 30: Aufnahme
- 32: Infusionsbeutel
- 34: Rollenklemme

## Patentansprüche

1. Medizinisches Infusionssystem mit
• mindestens einem zum Einlegen in oder Anschließen an einem Infusionsgerät (16), insbesondere einer Peristaltikpumpe oder einer Spritzenpumpe, vorgesehenen Einmalartikel (4), insbesondere einem Infusionsschlauch bzw. einer Spritze, der entweder selbst oder durch ein mit ihm verbundenes Zubehörteil mit einem maschinenlesbaren Code versehen ist, welcher eine Ablaufdauer, insbesondere eine Verweildauer oder eine Haltbarkeitsdauer oder ein Verfallsdatum, des Einmalartikels (4) beinhaltet oder repräsentiert,
• mindestens einem Infusionsgerät (16) mit einer Aufnahme (30) oder einem Anschluss für den Einmalartikel (4), ferner mit einem Sensor zur Erfassung des Codes, und ferner mit einer Pumpensteuerung (24), die derart ausgelegt oder programmiert ist, dass sie anhand des erfassten Codes die Ablaufdauer, insbesondere die Verweildauer, die Haltbarkeitsdauer oder das Verfallsdatum, des Einmalartikels (4) ermittelt.

2. Infusionssystem nach Anspruch 1, wobei die Pumpensteuerung (24) derart ausgelegt oder programmiert ist, dass sie anhand des erfassten Codes die Ablaufdauer, insbesondere die Verweildauer, die Haltbarkeitsdauer oder das Verfallsdatum, des Einmalartikels (4) ermittelt und mit einer Systemzeit vergleicht und je nach Ergebnis des Vergleichs ein Signal an den Benutzer erzeugt, das bevorzugt dazu geeignet ist einen Infusionsbetrieb mit dem Einmalartikel (4) zu blockieren oder zuzulassen und, bevorzugt im Fall einer Blockade, weiterbevorzugt das Signal als eine Warnmeldung über eine Benutzerschnittstelle ausgibt.

3. Infusionssystem nach Anspruch 1 oder 2, wobei der Einmalartikel (4) ein Infusionsschlauch (2) oder eine Spritze ist.

4. Infusionssystem nach einem der vorangehenden Ansprüche, wobei das Zubehörteil eine Schiebeklemme (10) ist.

5. Infusionssystem nach Anspruch 4, wobei das Infusionsgerät (16), insbesondere eine Infusionspumpe, eine Verschlussklappe (18) aufweist, die die Schiebeklemme (10) betätigt.

6. Infusionssystem nach einem der vorangehenden Ansprüche, wobei der Code in Form einer Farbkodierung (20) ausgebildet ist.

7. Infusionssystem nach Anspruch 6, wobei der Sensor ein Farbsensor (26) ist.

8. Infusionssystem nach einem der vorangehenden Ansprüche, wobei der Code ein graphischer Code ist oder durch ein RFID-Tag oder durch eine Ausformung verwirklicht ist.

9. Infusionssystem nach Anspruch 6, 7 oder 8, wobei in dem Infusionssystem, insbesondere in der Pumpensteuerung (24), eine Zuordnungstabelle (22) abgespeichert ist, die den Code mit einer Ablaufdauer, insbesondere einer Verweildauer, einer Haltbarkeitsdauer oder einem Verfallsdatum, verknüpft.

10. Infusionssystem nach Anspruch 9, wobei die Zuordnungstabelle (22) digital signiert und/oder verschlüsselt abgespeichert ist.

11. Infusionssystem nach einem der vorangehenden Ansprüche, wobei die Pumpensteuerung (24) derart ausgelegt oder programmiert ist, dass sie die Systemzeit mit einer externen Zeit synchronisiert.

12. Infusionssystem nach einem der vorangehenden Ansprüche, wobei die Pumpensteuerung (24) derart konfiguriert ist, dass ein autorisierter Benutzer, insbesondere nach Eingabe eines Berechtigungscodes, in der Lage ist, eine Blockade aufzuheben.

13. Infusionssystem nach einem der vorangehenden Ansprüche, wobei der Einmalartikel (4) ausgewählt ist aus der Gruppe umfassend Infusionsschlauch und Spritze.

14. Infusionssystem nach einem der vorangehenden Ansprüche, wobei das Infusionsgerät (16) ausgewählt ist aus der Gruppe umfassend Infusionspumpe, insbesondere Peristaltikpumpe und Spritzenpumpe,

15. Verfahren zum Betreiben eines medizinischen Infusionssystems, bevorzugt nach einem der vorangehenden Ansprüche, mit Überprüfung einer vorgegebenen Ablaufdauer, insbesondere einer Verweildauer, einer Haltbarkeitsdauer oder eines Verfallsdatums, eines zum Einlegen in oder Anschließen an eine Infusionsgerät (16) , insbesondere eine Infusionspumpe oder eine Spritzenpumpe, vorgesehenen Einmalartikels (4), der entweder selbst oder durch ein mit ihm verbundenes Zubehörteil mit einem maschinenlesbaren Code versehen ist, welcher die Ablaufdauer, insbesondere die Verweildauer, die Haltbarkeitsdauer oder das Verfallsdatum beinhaltet oder repräsentiert, wobei nach dem Einlegen des Einmalartikels (4) in oder Anschließen an ein Infusionsgerät (16)
• der Code durch einen Sensor erfasst wird,
• anhand des erfassten Codes die Ablaufdauer, insbesondere die Verweildauer, die Haltbarkeitsdauer oder das Verfallsdatum, des Einmalartikels (4) ermittelt wird und mit einer Systemzeit verglichen wird, und
• je nach Ergebnis des Vergleichs entweder ein Infusionsbetrieb mit dem Einmalartikel (4) blockiert oder zugelassen wird und bevorzugt
• ein Signal an den Benutzer erzeugt wird, das weiter bevorzugt dazu geeignet ist einen Infusionsbetrieb mit dem Einmalartikel (4) zu blockieren oder zu zulassen.
